(19) Europäisches Patentamt
European Patent Office
Office européen des brevets

(11) EP 0 686 616 B1

(12) **FASCICULE DE BREVET EUROPEEN**

(45) Date de publication et mention de la délivrance du brevet:
**15.10.1997 Bulletin 1997/42**

(51) Int. Cl.$^6$: **C07C 17/16**, C07C 19/01, C07C 19/03

(21) Numéro de dépôt: **95401215.9**

(22) Date de dépôt: **24.05.1995**

(54) **Procédé d'alcoolyse du tétrachlorure de carbone**
Verfahren zur Alkoholyse von Tetrachlorkohlenstoff
Process for alcoholysis of carbon tetrachloride

(84) Etats contractants désignés:
**BE DE ES FR GB IE IT NL**

(30) Priorité: **06.06.1994 FR 9406890**

(43) Date de publication de la demande:
**13.12.1995 Bulletin 1995/50**

(73) Titulaire: **ELF ATOCHEM S.A.**
**92800 Puteaux (FR)**

(72) Inventeurs:
- **Correia, Yves**
  **F-04160 Chateau Arnoux (FR)**
- **Fino, Noel**
  **F-04160 Chateau Arnoux (FR)**
- **Leduc, Philippe**
  **F-04600 Saint Auban (FR)**

(56) Documents cités:
**EP-A- 0 435 210**
**US-A- 2 172 568**
**US-A- 2 871 256**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 686 616 B1

Printed by Rank Xerox (UK) Business Services
2.14.17/3.4

## Description

La présente invention concerne un procédé d'alcoolyse du tétrachlorure de carbone ($CCl_4$) en présence d'une solution aqueuse d'un halogénure métallique.

La réaction d'alcoolyse du $CCl_4$ selon l'équation :

$$4ROH + CCl_4 \rightarrow 4RCl + 2H_20 + CO_2 \qquad (I)$$

conduit à des halogénures d'alkyle RCl et permet de valoriser le $CCl_4$, obtenu inévitblement lorsqu'on réalise notamment la fabrication des chlorométhanes supérieurs et dont l'utilisation sera interdite à la fin de ce siècle car il est fortement suspecté d'être responsable de la diminution de la couche d'ozone dans la stratosphère.

Dans le brevet français 793731 il est décrit un procédé d'alcoolyse du $CCl_4$ qui consiste à faire passer un mélange gazeux constitué d'un alcool (éthanol ou méthanol) et de $CCl_4$ sur des substances solides ayant une surface active telles que charbon actif ou gel de silice. Ces substances peuvent être éventuellement chargées de substances activantes telles que le chlorure de zinc ($ZnCl_2$) ou les composés du phosphore.

Dans la demande de brevet européen EP 435 210 il est décrit un procédé de préparation de $CH_3Cl$ qui consiste à faire passer un mélange gazeux constitué de $CH_3OH$ et de $CCl_4$ sur un lit catalytique solide de charbon actif contenant un oxyde ou un halogénure d'un élément choisi parmi les groupes I B, II A, II B, VI B, VII B et VIII de la classification périodique des éléments. La réaction se déroule à des températures comprises entre 150°C et 250°C sous des pressions égales à environ 10 $kg/cm^2$.

Dans le brevet allement DE 4 131 213 il est décrit un procédé d'alcoolyse du $CCl_4$ qui consiste à réaliser en phase gazeuse la réaction entre $CCl_4$ et un alcool aliphatique ayant de 1 à 10 atomes de carbone en présence d'un catalyseur solide qui est constitué d'alumine activée et/ou de silice.

On a maintenant trouvé un procédé d'alcoolyse du tétrachlorure de carbone qui consiste à envoyer simultanément, d'une part, du $CCl_4$ et d'autre part au moins un alcool ROH dans lequel R représente un radical alkyle linéaire ou ramifié ayant un nombre de carbone allant de 1 à 10 sur une composition catalytique, caractérisé en ce que ladite composition catalytique comprend une solution aqueuse d'un halogénure métallique.

Selon la présente invention, l'halogénure métallique est choisi parmi les halogénures des métaux des groupes IB (tel que le cuivre), II B (tels que le zinc et le cadminum), VI B (tels que le chrome et le molybdène), VIII (tels que le fer, cobalt et nickel) de la classification périodique des éléments.

Parmi ces halogénures métalliques on préfère utiliser les chlorures métalliques, et, tout particulièrement, le chlorure de zinc $ZnCl_2$.

A titre d'illustration d'alcools ROH utilisables selon la présente invention, on citera le méthanol, l'éthanol, le propanol, le butanol, l'isopropanol et le mélange d'au moins deux alcools précités.

La concentration pondérale en halogénure métallique de la composition catalytique est au plus égale à 98 % et, de préférence, comprise entre 50 % et 90 %.

La réaction peut être avantageusement réalisée sous une pression au plus égale à 10 bars et, de préférence, comprise entre 3 et 5 bars.

Selon la présente invention, la réaction peut être conduite à une température au plus égale à 250°C et, de préférence, comprise entre 150°C et 200°C.

Les réactifs peuvent être introduits dans la composition catalytique à l'état de vapeur et/ou à l'état liquide.

Le temps de séjour, calculé comme étant le rapport du volume total de la composition catalytique sur le volume total du mélange des réactifs gazeux par unité de temps dans les conditions de pression et de température telles que définies précédemment, est compris entre 10 et 60 secondes.

Le rapport molaire $ROH/CCl_4$ peut varier dans une large mesure. Il peut être inférieur, égal ou supérieur à la stoechiométrie de l'équation (I) selon que l'on souhaite avoir un excès de l'un ou de l'autre des réactifs.

La réaction d'alcoolyse du $CCl_4$ peut être également accompagnée d'une réaction équilibrée, simultanée de déshydratation de l'alcool ROH en éther dialkylique ROR, selon l'équation

$$2\ ROH \rightleftharpoons ROR + H_2O \qquad (II)$$

Selon la présente invention, cet équilibre peut être favorablement déplacée si l'on recycle l'éther formé avec le mélange des réactifs.

On ne sortirait pas du cadre de l'invention si on effectuait simultanément sur la même composition catalytique, et dans les mêmes conditions opératoires de pression et de température l'hydrochloration des alcools ROH selon l'équation :

$$ROH + HCl \rightarrow RCl + H2O \qquad (III)$$

Selon cette variante on préfère introduire le chlorure d'hydrogène sous forme gazeuse et dans une quantité telle qu'il se trouve en excès par rapport à la stoechiométrie de l'équation (III).

Cet excès peut varier dans une large mesure, il peut atteindre 30 % mais de préférence, est compris entre 10 % et 20 %.

Si l'on effectue simultanément l'alcoolyse de $CCl_4$ et l'hydrochloration des alcools, dans le rapport molaire

$$\frac{ROH}{CCl_4},$$

"ROH" représente le nombre de moles de ROH entrant

dans la réaction (I) plus le nombre de moles ROH entrant dans la réaction III.

Le procédé de la présente invention s'applique à la préparation des chlorures d'alkyle tels que $CH_3Cl$, $CH_3CH_2Cl$ et $CH_3(CH_2)_2Cl$ ou leur mélange.

Il s'applique tout particulièrement à la fabrication du $CH_3Cl$ par méthanolyse du $CCl_4$.

Selon la seconde variante du procédé on peut réaliser, simultanément à la méthanolyse du $CCl_4$ selon l'équation (I), l'hydrochloration du méthanol selon l'équation (III).

A la sortie de la composition catalytique la récupération des produits se fait par les moyens connus de l'homme du métier, à savoir détente, refroidissement puis une ou plusieurs distillation pour séparer les produits obtenus des réactifs non transformés qui, si besoin est, peuvent être recyclés.

Le procédé selon la présente invention, présente l'avantage de transformer quasi totalement $CCl_4$ en RCl sans formation gênante de sous-produits qui nécessiteraient des séparations coûteuses.

Les exemples qui suivent illustrent l'invention.

**EXEMPLE 1**

Dans un réacteur de laboratoire, en verre, de 150 ml, chauffé extérieurement par un bain d'huile, équipé d'un tube plongeur, d'un évent, d'une prise de température et d'une agitation magnétique, on place 50 ml d'une solution de chlorure de zinc à 90 % dans l'eau. La température du bain étant maintenue à 180°C et le réacteur étant sous 3,8 bars effectifs, on introduit en continu à 36 g/h, un mélange de méthanol et de $CCl_4$, (le temps de séjour est égal à 36 secondes) dans le rapport molaire $MeOH/CCl_4 = 4$ et on procède à l'analyse et à la mesure des gaz sortant du réacteur. On constate qu'à l'heure, on produit 19,8 g de chlorure de méthyle et 4,3 g de gaz carbonique, du $CCl_4$ et du méthanol non réagit étant présents en sortie. Les quantités évaluées correspondant à un taux de conversion du $CCl_4$ de 77 %.

**EXEMPLE 2**

Dans une unité industrielle de préparation de chlorure de méthyle par le procédé d'hydrochloration du méthanol par le chlorure d'hydrogène, on introduit, dans la composition catalytique constitué d'une solution aqueuse de $ZnCl_2$ à 70 %, à une température de 160-170°C, sous une pression de 3,5 bars, un mélange méthanol/tétrachlorure de carbone dans un rapport molaire de 95/5 et simultanément du chlorure d'hydrogène de manière à ce que celui-ci soit en excès stoechiométrique théorique de 13 % environ.

Le bilan entrée-sortie du réacteur industriel est le suivant :

| | Entrée (kg/h) | Sortie (Kg/h) |
|---|---|---|
| $CH_3OH$ | 4535 | 135 |
| $CCl_4$ | 1091 | 0 |
| HCl | 4737 | 752 |
| $H_2O$ | 0 | 2221 |
| $CH_3Cl$ | 0 | 6943 |
| $CO_2$ | 0 | 312 |

**Revendications**

1. Procédé d'alcoolyse du tétrachlorure de carbone qui consiste à envoyer simultanément, d'une part, du $CCl_4$ et d'autre part au moins un alcool ROH dans lequel R représente un radical alkyle linéaire ou ramifié ayant un nombre de carbone allant de 1 à 10 sur une composition catalytique, caractérisé en ce que ladite composition catalytique comprend une solution aqueuse d'un halogénure métallique choisi parmi les halogénures des métaux des groupes I B, II B, VI B, VIII de la classification périodique des éléments.

2. Procédé selon la revendication 1, caractérisé en ce que l'halogénure métallique est un chlorure métallique.

3. Procédé selon la revendication 2, caractérisé en ce que le chlorure métallique est le chlorure de zinc $ZnCl_2$.

4. Procédé selon l'une des revendications 1 à 3, caractérisé en ce que la concentration pondérale en halogénure métallique de composition catalytique est au plus égale à 98 %.

5. Procédé selon la revendication 4, caractérisé en ce que la concentration pondérale en halogénure métallique de la composition catalytique est comprise entre 50 % et 90 %.

6. Procédé selon l'une des revendications 1 à 5, caractérisé en ce que la réaction est conduite à température au plus égale à 250°C.

7. Procédé selon la revendication 6, caractérisé en ce que la réaction est conduite à une température comprise entre 150°C et 200°C.

8. Procédé selon l'une des revendications 1 à 7 caractérisé en ce que la réaction est réalisée sous une

pression au plus égale à 10 bars.

**9.** Procédé selon la revendication 8, caractérisé en ce que la réaction est réalisée sous une pression comprise entre 3 et 5 bars.

**10.** Procédé selon l'une des revendications 1 à 9, caractérisé en ce qu'on effectue, simultanément à l'alcoolyse du tétrachlorure de carbone, l'hydrochloruration des alcools ROH par introduction de chlorure d'hydrogène sur la composition catalytique.

**11.** Procédé selon l'une des revendications 1 à 10, caractérisé en ce que l'alcool est le méthanol.

## Claims

**1.** Process for the alcoholysis of carbon tetrachloride which consists in simultaneously sending, on the one hand, $CCl_4$ and, on the other hand, at least one alcohol ROH in which R represents a linear or branched alkyl radical having a member of carbon atoms ranging from 1 to 10, over a catalytic composition, characterized in that the said catalytic composition comprises an aqueous solution of a metal halide chosen from halides of the metals of groups I B, II B, VI B and VIII of the Periodic Table of the Elements.

**2.** Process according to Claim 1, characterized in that the metal halide is a metal chloride.

**3.** Process according to Claim 2, characterized in that the metal chloride is zinc chloride, $ZnCl_2$.

**4.** Process according to one of Claims 1 to 3, characterized in that the weight concentration of metal halide in the catalytic composition is at most equal to 98 %.

**5.** Process according to Claim 4, characterized in that the weight concentration of metal halide in the catalytic composition is between 50 % and 90 %.

**6.** Process according to one of Claims 1 to 5, characterized in that the reaction is carried out at a temperature at most equal to 250°C.

**7.** Process according to Claim 6, characterized in that the reaction is carried out a temperature between 150°C and 200°C.

**8.** Process according to one of Claims 1 to 7, characterized in that the reaction is performed at a pressure at most equal to 10 bar.

**9.** Process according to Claim 8, characterized in that the reaction is performed at a pressure between 3 and 5 bar.

**10.** Process according to one of Claims 1 to 9, characterized in that the alcoholysis of carbon tetrachloride and the hydrochlorination of the alcohols ROH are carried out simultaneously by introduction of hydrogen chloride onto the catalytic composition.

**11.** Process according to one of Claims 1 to 10, characterized in that the alcohol is methanol.

## Patentansprüche

**1.** Verfahren zur Alkoholyse von Kohlenstofftetrachlorid, bei dem man gleichzeitig $CCl_4$ einerseits und mindestens einen Alkohol ROH andererseits über eine katalytische Zusammensetzung schickt, wobei R einen linearen oder verzweigten Rest mit einer Kohlenstoffanzahl von 1 bis 10 darstellt, dadurch gekennzeichnet, daß die katalytische Zusammensetzung eine wäßrige Lösung eines Metallhalogenids umfaßt, das aus Metallhalogeniden der Gruppe IB, IIB, VIB und VIII des Periodensystems der Elemente ausgewählt ist.

**2.** Verfahren nach Anspruch 1, dadurch gekennzeichnet, daß das Metallhalogenid ein Metallchlorid ist.

**3.** Verfahren nach Anspruch 2, dadurch gekennzeichnet, daß das Metallchlorid Zinkchlorid $ZnCl_2$ ist.

**4.** Verfahren nach einem der Ansprüche 1 bis 3, dadurch gekennzeichnet, daß die Gewichtskonzentration der katalytischen Zusammensetzung an Metallhalogenid höchsten 98 % beträgt.

**5.** Verfahren nach Anspruch 4, dadurch gekennzeichnet, daß die Gewichtskonzentration der katalytischen Zusammensetzung an Metallhalogenid zwischen 50 % und 90 % liegt.

**6.** Verfahren nach einem der Ansprüche 1 bis 5, dadurch gekennzeichnet, daß die Reaktion bei einer Temperatur von höchstens 250 °C durchgeführt wird.

**7.** Verfahren nach Anspruch 6, dadurch gekennzeichnet, daß die Reaktion bei einer Temperatur zwischen 150 °C und 200 °C durchgeführt wird.

**8.** Verfahren nach einem der Ansprüche 1 bis 7, dadurch gekennzeichnet, daß die Reaktion unter einem Druck von höchstens 10 bar durchgeführt wird.

**9.** Verfahren nach Anspruch 8, dadurch gekennzeichnet, daß die Reaktion unter einem Druck zwischen 3 und 5 bar durchgeführt wird.

**10.** Verfahren nach einem der Ansprüche 1 bis 9, dadurch gekennzeichnet, daß man gleichzeitig mit

der Alkoholyse des Kohlenstofftetrachlorids die Hydrochlorierung der Alkohole ROH an der katalytischen Zusammensetzung durch Zugabe von Chlorwasserstoff durchführt.

**11.** Verfahren nach einem der Ansprüche 1 bis 10, dadurch gekennzeichnet, daß der Alkohol Methanol ist.